# EUROPÄISCHE PATENTSCHRIFT

(11) **EP 0 594 034 B1**
(45) Veröffentlichungstag und Bekanntmachung des Hinweises auf die Patenterteilung: **08.01.1997**
(21) Anmeldenummer: 93116468.5
(22) Anmeldetag: 12.10.1993
(51) Int. Cl.: A61F 13/00, A61M 35/00

(54) **Plastisch verformbare Kompresse**
Plastically-deformable compress
Compresse à déformation plastique

(30) Priorität: 22.10.1992 CH 3272/92
(43) Veröffentlichungstag der Anmeldung: 27.04.1994
(73) Patentinhaber: IVF INTERNATIONALE VERBANDSTOFF-FABRIK SCHAFFHAUSEN, CH-8212 Neuhausen am Rheinfall (CH)
(72) Erfinder: Dudler, Markus, CH-8248 Uhwiesen (CH); De Couet, Alexandre, CH-6343 Risch (CH); Pochon, Jean-Pierre, Dr.med., CH-8124 Maur (CH)
(74) Vertreter: Blum, Rudolf Emil Ernst

(56) Entgegenhaltungen:
- EP-A- 0 031 018
- EP-A- 0 120 710
- EP-A- 0 342 927
- WO-A-90/01913
- GB-A- 1 192 581
- GB-A- 2 163 356
- DATABASE WPI Week 8829, Derwent Publications Ltd., London, GB; AN 88-205296 & WO-A-88 04923 (BRITISH TECHNOLOGY GROUP) 14. Juli 1988
- DATABASE WPI Week 9301, Derwent Publications Ltd., London, GB; AN 93-003859 & JP-A-04 331 144 (TOPPAN PRINTING) 19. November 1992 & PATENT ABSTRACTS vol. 017, no. 172 (M-1392) 2. April 1993 & JP-A-04 331 144 (TOPPAN PRINTING) 19. November 1992

## Beschreibung

Die Erfindung betrifft eine Kompresse gemäss dem Oberbegriff des ersten Patentanspruchs.

Die Behandlung von Wunden in einem feuchten Milieu ist seit langer Zeit als Verbandstechnik bekannt und wird in vielen Fällen angewendet. Bei der Behandlung mit Feuchtverbänden wird die Wunde mit einem feuchten Verband abgedeckt, der zum Beispiel mit einer physiologischen Lösung getränkt ist. Insbesondere eignet sich dieses Vorgehen für die Behandlung von Problemwunden, wie zum Beispiel Ulcera oder Decubitus. Auch im Bereich der kosmetischen Chirurgie und für die Behandlung von Brand-, Schürf- und Schnittwunden findet die Feuchtverband-Technik Anwendung.

Bisher wurden Feuchtverbände meist so erstellt, dass Verbandstoff mit physiologischer Lösung getränkt und unter einem Deckverband auf die Wunde gelegt wurde. Dieses Vorgehen ist aber aus verschiedenen Gründen nachteilig. So trocknet das befeuchtete Material innert weniger Stunden aus. Es kann zu einer mechanischen Reizung der Wunde kommen und es besteht die Gefahr, dass sich sogenannte Feuchtkammern bilden, d.h. mit Exsudat ausgefüllte Zwischenräume zwischen dem Verband und dem Wundgewebe. Solche Feuchtkammern können z.B. als Infektionsherde zu weiteren Problemen führen. Auch bleiben die konventionellen Feuchtverbände bisweilen an den Wunden kleben, was zu einem schmerzhaften, traumatischen Verbandwechsel führt.

Eine Kompresse gemäss Oberbegriff von Anspruch 1 ist aus EP-A-0 031 018 bekannt.

In EP-A-0 031 018, EP-A-0 342 927 und WO 90/01913 werden Kompressen beschrieben, bei welchen ein Polymerisat resp. ein Superabsorber zur Wasserspeicherung verwendet wird. Das Polymerisat resp. der Superabsorber ist von einem gegebenenfalls elastischen Gewebe, einem Vlies, einer porösen Folie oder einem Schaumstoff umhüllt. Eine derartige Kompresse bleibt relativ lang feucht. Eine solche Kompresse erlaubt es, dank der Verwendung eines Superabsorbers als Feuchtigkeitsspeicher eine volumenbezogen grosse Flüssigkeitsmenge zu speichern. Ausserdem wird die Feuchtigkeit gleichmässig abgegeben, so dass der Verband lange Zeit feucht bleibt und während längerer Zeit nicht ausgewechselt zu werden braucht. Es zeigt sich jedoch, dass auch hier Feuchtkammern zwischen der Kompresse und der Wunde entstehen, insbesondere wenn das Wundprofil unregelmässig ist. Deshalb müssen auch diese Kompressen oft gewechselt und die Wunde sorgfältig gereinigt werden.

Deshalb stellt sich die Aufgabe, ein Verbandmittel dieser Art bereitzustellen, das keine mechanische Reizung der Wunde verursacht, die Bildung von Feuchtkammern verhindert, auch bei tieferen Wunden stets mit dem Wundgrund in Kontakt bleibt und mit der Wunde nicht verklebt.

Diese Aufgabe wird mittels der Kompresse gemäss der Lehre im Kennzeichen des ersten Patentanspruchs gelöst

Im Gegensatz zu den bekannten Ausführungen nach EP-A-0 031 018 weist die erfindungsgemässe Kompresse eine Hülle auf, die mindestens teilweise aus einer textilen Fläche besteht, welche in Längs-, Quer- und Diagonalrichtung nicht elastisch dehnbar ist, wodurch die Kompresse plastisch verformbar ist und sich auch dem Wundprofil tiefer Wunden gut anpasst. Im Gegensatz zu einer nicht dehnbaren oder elastisch verformbaren Kompresse, folgt die erfindungsgemäss plastisch verformbare Kompresse allen Unregelmässigkeiten, die Wunde bleibt gleichmässig feucht und Feuchtkammern werden verhindert. Sie übt nur geringe Kräfte auf die Wunde aus, so dass keine mechanische Reizung verursacht wird. Eine sehr gute plastische Verformbarkeit wird dadurch erreicht, dass die Hülle der Kompresse aus einem nicht-elastisch dehnbaren Material, vorzugsweise aus einem Gestrick resp. Gewirk besteht. Damit kann die Hülle in einer oder mehreren Richtungen gedehnt oder verformt werden, ohne dass sie sich von selbst wieder zusammenzieht resp. ausrichtet. Die Oberfläche einer derartigen Kompresse kann z.B. einfach lokal ausgebeult werden und schmiegt sich dem Wundprofil gut an. Der auf der Wunde aufliegende Teil der Kompresse verschiebt sich bei mechanischer Beanspruchung (parallel zur Wundoberfläche) nicht gegenüber der Wunde.

Vorzugsweise besteht die Hülle aus zwei Lagen eines thermoplastischen Materials, zwischen denen der Absorber angeordnet ist. Die Lagen sind längs ihren Rändern durch Schweissnähte verschweisst. Verlaufen die Schweissnähte einer rechteckigen Kompresse schief zu den Maschen- bzw. Fadenreihen der Hülle, so sind die Nähte sehr gleichmässig und ein Ausfransen der Hülle wird verhindert.

Die Kompresse kann in einer ersten Variante in feuchtem Zustand mit gequollenem Absorber wasserdampfdicht verpackt und gebrauchsfertig angeboten werden. In einer zweiten Variante wird sie trocken gelagert und erst vor Gebrauch vom Anwender befeuchtet.

Es zeigt sich, dass die erfindungsgemässe Kompresse über mindestens 24 Stunden, ja sogar bis zu 72 Stunden feucht bleibt und nicht mit der Wunde verklebt. Sie bewirkt keine Hautreaktionen und ermöglicht eine Wundreinigung, indem abgestossenes, totes Gewebe beim Wechsel grösstenteils an der Kompresse kleben bleibt. Sie verhindert auch den Rückstau von Gewebeflüssigkeit unter dem Verband. Selbst tiefe Wunden werden problemlos feucht gehalten. Die Kompresse lässt sich atraumatisch entfernen. Ausserdem wird die Quellflüssigkeit im Superabsorber zurückgehalten und läuft nicht spontan aus. Zudem können in der Quellflüssigkeit Wirkstoffe gelöst sein. Insgesamt schafft die Kompresse ein Wundmilieu, welches den Heilungsprozess unterstützt.

Durch eine Hydrophobierung der Hülle, z.B. mit Wachs, oder durch Wahl eines nicht-saugenden Hüllenmaterials wird der Austausch von Flüssigkeit vereinfacht. Insbesondere kann Körperflüssigkeit einfach in das Innere der Kompresse gelangen. Bei herkömmlichen Lösungen mit hydrophilen Hüllen sammelt sich Körperflüssigkeit in der Hülle und kann dort z.B. koagulieren. Dadurch wird die Hüllendurchlässigkeit beeinträchtigt und ausserdem kann die Hülle mit der Wunde verkleben. Bei einer hydrophoben und/oder nicht-saugenden Hülle tritt eine allfällige Koagulation erst im Inneren der Kompresse und über einen grösseren Volumenbereich verteilt auf, wodurch diese Probleme vermieden werden.

Die Kompresse kann verschiedenste Wirk- und Hilfsstoffe aufnehmen. Durch Zusatz von geruchsverhindernden und/oder geruchsmaskierenden Hilfsstoffen, wie z.B. Stoffe zur Hemmung des Bakterienwachstums (z.B. quarternäre Amine) oder aetherische Oele (z.B. Eucalyptusoel), ergeben sich insbesondere bei der Behandlung von übelriechenden Wunden (z.B. schweren Fällen von Decubitus) bessere Behandlungsbedingungen.

Auch ist es möglich, eine geeignete Wirkstoffe enthaltende Kompresse auf eine nicht verletzte Körperoberfläche aufzubringen. Die Wirkstoffe sind in diesem Falle so gewählt, dass sie durch die Haut dringen und so ihre Wirkung entfalten können.

Weitere Vorteile und Anwendungen der erfindungsgemässen Kompresse ergeben sich aus der folgenden Beschreibung einer Ausführung der Erfindung anhand der Figuren. Dabei zeigen:
Figur 1 den prinzipiellen Aufbau einer erfindungsgemässen Kompresse im Schnitt;
Figur 2 einen Schnitt durch eine Kompresse auf einer Wunde;
Figur 3 das Abrollen der Kompresse bei horizontalen Kräften an der Aussenseite;
Figur 4 einen Schnitt durch eine verpackte Kompresse;
Figur 5 und 6 Kompressen mit zwei Kompartimenten;
Figur 7 eine Kompresse mit eingeschlossenen Wirkstoffzellen;
Figur 8 und 9 Kompressen mit nur teilweise durchlässiger Hülle; und
Figur 10 eine schematische mikroskopische Ansicht des Hüllenmaterials.

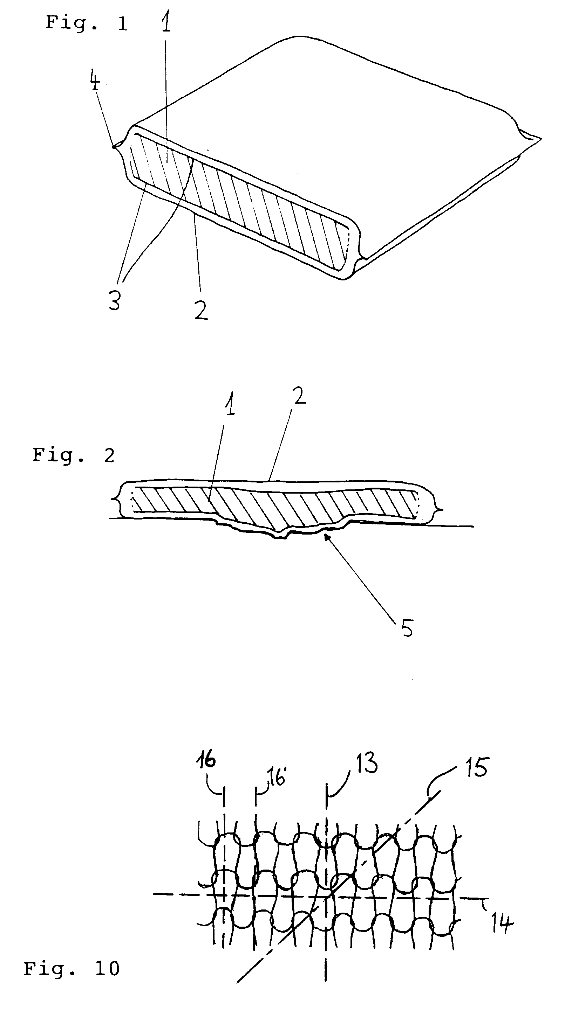

Der prinzipielle Aufbau der erfindungsgemässen Kompresse ist aus der Schnittansicht von Figur 1 ersichtlich. Sie besteht im wesentlichen aus einem feuchten Quellkörper 1, der von einer flüssigkeitsdurchlässigen Hülle 2 umgeben ist.

Der Quellkörper 1 besteht aus einem superabsorbierenden Material, vorzugsweise in Laminatform. Er kann aus mehreren Laminaten zusammengesetzt sein. Das superabsorbierende Laminat in diesem Ausführungsbeispiel hat eine Beladung von zwischen 60 und 80 g/m² an Polymer und eine Flüssigkeitsaufnahmekapazität von ca. 3500 g/m² 0.9%-ige NaCl-Lösung. Im gequollenen Zustand ist es von gelartiger Konsistenz. Dies führt zu einer Kompresse, die leicht verformbar und plastisch anschmiegsam ist. Ober- und unterhalb des Superabsorbers ist je eine Laminatschicht 3 aus Zellulose angeordnet. Diese Schichten fixieren den Superabsorber und gewährleisten dessen gleichmässige Verteilung. Die Laminat-Ober- und Unterseiten 3 einerseits und die Hülle 2 andererseits verhindern ausserdem, dass der gequollene Superabsorber direkt mit der Wunde in Kontakt kommt.

Der Superabsorber ist mit einer geeigneten Flüssigkeit, wie z.B. einer physiologischen Kochsalzlösung (0.9 %) getränkt. Der Absorber wird vorzugsweise nur soweit gesättigt, dass die Kompresse bei leichtem Zusammendrücken nicht tropft. Im Ausführungsbeispiel von 10 cm x 10 cm erreicht die erfindungsgemässe Kompresse mit 54 ml Flüssigkeit ihre optimale Quellung und hat gebrauchsfertig eine Dicke zwischen 5 und 10 mm.

Die Hülle 2 besteht aus einem wasserdurchlässigen Material. Sie ermöglicht also den Austritt von Feuchtigkeit aus dem feuchten Quellkörper 1.

In der vorliegenden Ausführung wird als Hülle ein Gestrick auf Polypropylen-Basis verwendet. Das Gestrick besteht aus zwei Lagen, von denen eine die Unterseite und die andere die Oberseite der Kompresse bildet, und die auf allen Seiten fest miteinander verschweisst sind. Das Gestrick weist im entspannten Zustand zwischen 10 und 40 Maschenreihen pro cm und Maschenhöhen zwischen 0.2 und 2 mm auf. Bei einer Deformation der Kompresse entsteht eine Umorientierung der Maschen solcher textilen Flächengebilde derart, dass sie ihre räumliche Ausdehnung bleibend verändert.

Als Garn wird bei der Hülle ein luftblastexturiertes Multifilament aus reinem Polypropylen verwendet, vorzugsweise mit einer Fadenstärke von 170 dtex mit 80 Fibrillen oder bis zu 85 dtex mit 40 Fibrillen (z.B. Atrex der Arova Schaffhausen AG, Schaffhausen Schweiz). Dank einer vorzüglichen Permeabilität wird Flüssigkeit sehr rasch durch die Hülle hindurch zur Wunde oder von der Wunde her in den Quellkörper hinein abgeleitet, wobei das Fadenmaterial kaum Feuchtigkeit (maximal etwa 0.1 Gewichts-%) aufnimmt.

Das verwendete Gestrick oder Gewirk hat gegenüber den meisten anderen Flächengebilden den Vorteil, dass es in allen Richtungen plastisch verformbar ist. Wie in Figur 2 gezeigt wird, kann damit erreicht werden, dass die ganze Kompresse sich dem Profil der Wunde 5 optimal anpasst. Selbst tiefe Wunden werden vom Verband ausgefüllt. Die Kompresse ist form- bzw. modellierbar und geht, einmal geformt, nicht von selbst in ihre Ausgangslage zurück, da die Verformung plastisch und nicht elastisch ist.

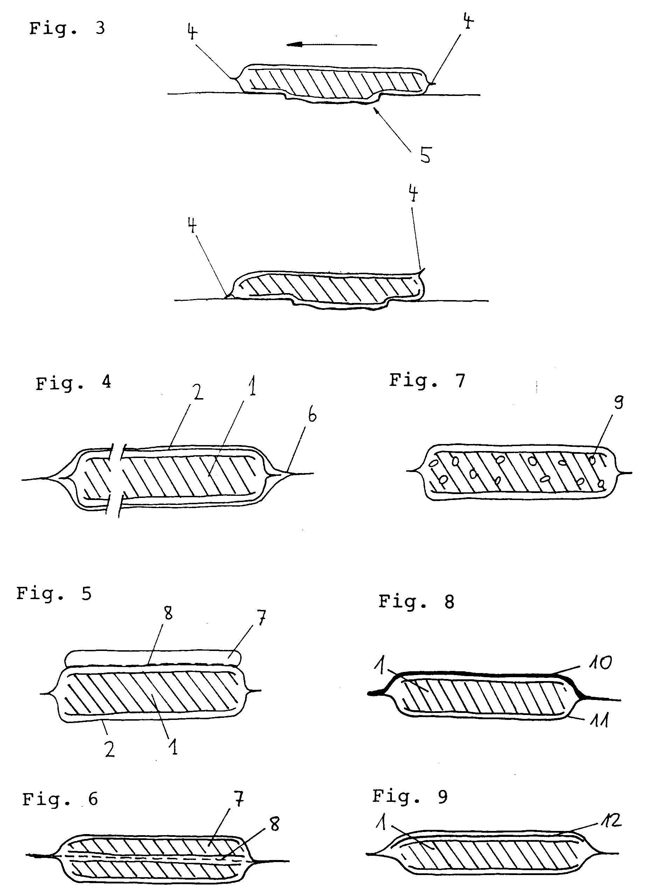

Die aus ihrem Aufbau resultierende Konsistenz der Kompresse hat weiter den Vorteil, dass vom darüberliegenden Verband an der Kompresse angreifende mechanische Scherkräfte nicht auf die Wunde übertragen werden. Insbesondere die oft parallel zur Wundoberfläche auftretenden Kräfte am aussenliegenden Verband führen nicht dazu, dass die Kompresse gegenüber der Wunde verschoben wird, sondern bewirken ein "Abrollen" der Kompresse, wie es in Figur 3 schematisch gezeigt wird. Eine durch einen Pfeil angedeutete Kraft, welche zum Beispiel durch ein geringfügiges Verrutschen des Verbandes bewirkt wird, führt zu einer Verschiebung der oberen Kompressenfläche, ohne dass der unterseitige Kompressenteil dabei verrutscht. Dadurch werden Reizungen der Wunde, Neuverletzungen und Schmerzen vermieden.

Wie bereits erwähnt wurde, sind die obere und untere Lage der Hülle miteinander verschweisst. Hierbei sind die Maschen- bzw. Fadenreihen 13, 14 (vgl. Fig. 10) so orientiert, dass die Schweissnähte nicht parallel dazu verlaufen. Vorzugsweise wird der Winkel zwischen den Maschen- und Fadenreihen 13, 14 und den Schweissnähten in einem Bereich von etwa 2 bis 45 Grad gewählt. Eine mögliche Schweissrichtung wird in Figur 10 durch die strichpunktierte Linie 15 angedeutet.

Dadurch wird erreicht, dass zur Schweissung eine gleichmässige, wohldefinierte Menge Hüllenmaterial zur Verfügung steht. Dies ist bei einer parallel zu den Maschen- resp. Fadenreihen verlaufenden Schweissnaht nicht der Fall: Eine Schweissnaht entlang Linie 16 der Figur 10 erfasst weniger Hüllenmaterial als eine Schweissnaht entlang Linie 16'. Ausserdem wird durch eine schräg verlaufende Schweissnaht ein Ausfransen der Fäden des Hüllenmaterials verhindert.

Wie eingangs erläutert, wird durch die Verwendung eines Gestricks oder Gewirks, wie es z.B. in Figur 10 angedeutet ist, die nötige plastische Verformbarkeit der Kompresse erreicht. Das verwendete Gestrick ist in Längs-, Quer- und Diagonalrichtung plastisch dehnbar resp. stauchbar und kann sich einer Ausformung der Kompresse optimal anpassen.

Das Gestrickmaterial auf Polypropylenbasis ist hydrophob. Wird ein hydrophiles Textilmaterial verwendet, so kann dieses z.B. mittels Wachs hydrophobiert werden, um die eingangs erwähnten, vorteilhaften Eigenschaften zu erreichen.

Figur 4 zeigt eine bevorzugte Verpackung der erfindungsgemässen Kompresse. Dabei ist die bereits mit Flüssigkeit versehene, gequollene Kompresse 1,2 gebrauchsfertig in einem wasserdichten, verschweissten Kunststoffbeutel 6 verpackt. In dieser Verpackung ist die gebrauchsfertige, feuchte Kompresse mehrere Jahre haltbar.

Zur Anwendung wird der Beutel 6 geöffnet, die feuchte Kompresse entnommen und auf die Wunde gelegt. Da in der hier beschriebenen Ausführung Ober- und Unterseite der Kompresse gleich ausgeführt sind, braucht dabei nicht darauf geachtet zu werden, welche Seite der Kompresse auf die Wunde zu liegen kommt. Dies vereinfacht die Anwendung.

Zur Fixierung der Kompresse wird vorzugsweise eine elastische, komprimierende, nicht aufsaugende Binde verwendet.

Die Kompression unterstützt den kontinuierlichen Kontakt der Kompresse mit dem Wundgrund.

Die erfindungsgemässe Kompresse schafft ideale Bedingungen für einen natürlichen Wundheilungsprozess. Die im Quellkörper gespeicherte Flüssigkeit wird kontinuierlich an die Wunde abgegeben.

Die Flüssigkeit, z.B. physiologische Kochsalzlösung, gewährt durch die Isotonie den Granulationszellen des Hautepithels die richtige Ionenkonzentration. Die Wunde wird so gegen Feuchtigkeits- und Ionenverlust geschützt. Die Flüssigkeitsaufnahme und -abgabe der regenerierenden Hautzellen wird ebenfalls reguliert und dadurch der Heilungsprozess optimal unterstützt. Durch das feuchte Milieu an der Wunde verklebt diese auch nicht mit der Kompresse. Ausserdem ist die Kompresse luftdurchlässig.

Das oben beschriebene bevorzugte Ausführungsbeispiel kann in vielen Punkten variiert und den jeweiligen Bedürfnissen angepasst werden.

So ist zum Beispiel in einer weiteren Ausführung der Quellkörper nicht mit reiner physiologischer Kochsalzlösung getränkt, sondern mit einer Mischung aus physiologischer Kochsalzlösung und einem Desinfektionsmittel, z.B. PVP-Jod. Sie kann auch einen desodorierenden Geruchsstoff, insbesondere ein ätherisches Oel enthalten. Je nach Anwendung sind weitere Hilfsstoffe oder Wirkstoffe denkbar.

Ebenfalls ist es möglich, eine Kompresse mit transdermal wirkenden Wirkstoffen auf die unverletzte Haut aufzubringen. In diesem Falle wirkt die Kompresse als Depot für die transdermale Applikation dieser Wirkstoffe und liegt ebenfalls vollflächig auf der intakten Haut auf. Die Wirkstoffe werden dabei kontinuierlich an die Haut abgegeben. In einer bevorzugten Ausführung wird in diesem Falle zur Regelung der Abgabe eine Hülle mit selektiver oder beschränkter Permeabilität verwendet, wie sie dem Fachmann bekannt ist.

Die Grösse und Form der Kompressen kann den jeweiligen Erfordernissen angepasst werden. In den vorliegenden Ausführungsbeispielen sind die Kompressen quadratisch mit typischen Massen von 7.5 cm x 7.5 cm oder 10 cm x 10 cm und einer typischen Dicke zwischen 2 und 10 mm. Selbstverständlich sind aber auch rechteckige, runde, ovale oder andere Ausführungen denkbar.

Zur besseren Speicherung von Flüssigkeit und/oder Wirkstoffen und zur Steuerung ihrer Abgabe ist es auch denkbar, eine erfindungsgemässe Kompresse aus mehreren Kompartimenten zu formen, wie sie in den Figuren 5 und 6 gezeigt werden. In Figur 5 ist auf der Oberseite der Kompresse eine Tasche 7 angebracht. Diese kann z.B. ebenfalls aus einem umhüllten Quellkörper bestehen, welcher mit einem besonderen Wirkstoff getränkt ist. Im Gegensatz zum Rest der Kompresse kann diese Tasche aber z.B. hermetisch abgeschlossen sein und somit ihren Inhalt schützen. Die Trennschicht 8 ist deshalb so ausgebildet, dass sie permeabel wird, wenn die Kompresse auf der Wunde oder Haut aufliegt, indem sie z.B. unter Körperwärme oder pH-abhängig durchlässig wird. Alternativ kann sie auch mechanisch aufbrechbar sein. Figur 6 zeigt eine alternative Ausführungsform einer derartigen Kompresse, welche im wesentlichen symmetrisch aufgebaut ist. Hier ist die Trennschicht 8 zentral angeordnet.

In einer anderen Ausführungsform kann das obere Kompartiment 7 auch einen Quellkörper aufweisen, der mit der gleichen Flüssigkeit wie der Quellkörper 1 des unteren Kompartiments getränkt ist. Die Trennschicht 8 ist dann so ausgeführt, dass sie erst nach mehreren Stunden der Anwendung durchlässig wird. Damit tritt eine verzögerte Feuchtigkeitsabgabe aus dem oberen Kompartiment 7 auf, was die Aktivzeit (Feuchthaltezeit) der Kompresse erhöht.

Es ist auch denkbar, dass das obere Kompartiment 7 keinen Quellkörper enthält, sondern nur einen Wirkstoff. Dieser kann dann insbesondere in einer nichtwässrigen Form aufbewahrt werden, was je nach Wirkstoff längere Lagerzeiten erlaubt. Wird nun die Trennwand 8 durchlässig oder wird sie durchlässig gemacht, so geht der Wirkstoff in das untere Kompartiment über und kann seine Wirkung entfalten.

In diesen Mehrkompartiments-Ausführungen kann selbstverständlich die Grösse der Kompartimente sowie ihre Anzahl und Lage vielfältig variiert werden. Insbesondere können die Kompartimente auch als kleine Taschen 9 im Inneren des Kompressenkörpers ausgeführt sein, wie es aus Figur 7 ersichtlicht ist.

In den Figuren 8 und 9 sind weitere Ausführungen der erfindungsgemässen Kompresse gezeigt. In Figur 8 ist die Hülle nur in einem unteren Bereich 11 feuchtigkeitsdurchlässig gestaltet, während die Kompresse an ihrer Oberseite mit einer feuchtigkeitsundurchlässigen Oberfläche 10 versehen ist. Dadurch wird verhindert, dass ein Teil der Feuchtigkeit nach oben abgegeben und vom Fixationsverband aufgenommen wird und die Aktivzeit (Feuchthaltezeit) der Kompresse erhöht. Figur 9 zeigt eine alternative Ausführung einer derartigen Kompresse, bei der eine feuchtigkeitsundurchlässige Lage 12 oberhalb des Quellkörpers im Innern der Kompresse angeordnet ist.

Vorzugsweise steht die Kompresse in feuchtem, gequollenem Zustand in einer geeigneten Packung zur Verfügung, wie sie z.B. oben beschrieben wird. Es ist allerdings auch denkbar, dass die Kompresse in Trockenform gelagert und erst unmittelbar vor der Verwendung befeuchtet wird. Damit verringern sich Verpackungs- und Transportkosten sowie das Transport- und das Lagervolumen. In diesem Fall kann die Kompresse ausserdem mit einem Wirkstoff versehen werden, der in feuchtem Zustand nur beschränkt lagerbar ist. Dieser Wirkstoff wird in Trockenform in die Kompresse eingebracht und kommt erst beim Tränken des Quellkörpers mit Flüssigkeit in Berührung.

## Patentansprüche

1. Feuchtverband-Kompresse zur Wundbehandlung im feuchten Milieu, welche einen Quellkörper (1) aufweist, welcher in einer mindestens teilweise feuchtigkeitsdurchlässigen Hülle (2) angeordnet ist, wobei der Quellkörper (1) zur Speicherung und kontinuierlichen Abgabe einer Flüssigkeit einen Superabsorber enthält oder aus einem Superabsorber besteht, der in gequollenem Zustand von gelartiger Konsistenz ist, dadurch gekennzeichnet, dass die Hülle (2) mindestens teilweise aus einer textilen Fläche besteht, welche in Längs-, Quer- und Diagonalrichtung nicht-elastisch dehnbar ist, derart, dass die Kompresse im feuchten Zustand weich und plastisch verformbar ist.

2. Kompresse nach Anspruch 1, dadurch gekennzeichnet, dass die Hülle (2) mindestens teilweise aus einem Gewirk oder einem Gestrick besteht.

3. Kompresse nach Anspruch 2, dadurch gekennzeichnet, dass die Hülle aus einem Gewirk oder Gestrick aus reinem Polypropylen besteht.

4. Kompresse nach Anspruch 3, dadurch gekennzeichnet, dass das Gewirk oder Gestrick aus einem Garn besteht, welches ein luftblastexturiertes Multifilament ist.

5. Kompresse nach Anspruch 4, dadurch gekennzeichnet, dass das Garn eine Fadenstärke von 170 dtex mit 80 Fibrillen oder bis zu 85 dtex mit 40 Fibrillen aufweist.

6. Kompresse nach einem der vorangehenden Ansprüche, dadurch gekennzeichnet, dass die Hülle (2) aus zwei Lagen eines thermoplastischen Materials besteht, die den Quellkörper umschliessen und längs ihren Rändern durch Schweissnähte verschweisst sind.

7. Kompresse nach einem der Ansprüchen 2 - 5 und nach Anspruch 6, dadurch gekennzeichnet, dass sie rechteckig ist und dass die Schweissnähte gegenüber den Maschen- oder Fadenreihen des Gewirks resp. des Gestricks einen Winkel zwischen 2 und 45 Grad bilden.

8. Kompresse nach einem der vorangehenden Ansprüche, dadurch gekennzeichnet, dass die Hülle aus einem nicht saugenden und/oder hydrophobierten Material besteht.

9. Kompresse nach einem der vorangehenden Ansprüche, dadurch gekennzeichnet, dass sie einen desodorierenden Hilfsstoff enthält.

10. Kompresse nach einem der vorangehenden Ansprüche, dadurch gekennzeichnet, dass der Quellkörper (1) zur Gewährleistung einer gleichmässigen Verteilung des Superabsorbers zwischen zwei Laminatschichten (3) angeordnet ist.

11. Kompresse nach einem der vorangehenden Ansprüche, dadurch gekennzeichnet, dass sie mehrere, durch Trennschichten (8) unterteilte Kompartimente aufweist.

12. Kompresse nach Anspruch 11, dadurch gekennzeichnet, dass die Trennschichten (8) durch mechanischen, chemischen und/oder thermischen Einfluss mindestens teilweise durchlässig machbar sind.

## Claims

1. Moist bandage compress for wound treatment in a moist environment comprising a swelling body (1), which is arranged in an at least partially humidity permeable cover (2), wherein the swelling body (1) comprises a or consists of a superabsorbent for storing and continuously releasing a liquid, which, in its swollen state, is of gel like consistency, characterised in that the cover (2) consists at least partially of a textile area which can be stretched non-elastically in longitudinal, transversal and diagonal directions, such that the compress is soft and plastically deformable in its moist state.

2. Compress of claim 1 characterised in that the cover (2) consists at least partially of a knit fabric.

3. Compress of claim 2 characterised in that the cover consists of a knit fabric of pure polypropylene.

4. Compress of claim 3 characterised in that the knit fabric consists of a yarn that is a air-flow textured multifilament.

5. Compress of claim 4 characterised in that the yarn has a thread thickness of 170 dtex with 80 fibres or up to 85 dtex with 40 fibres.

6. Compress of one of the preceding claims characterised in that the cover (2) consists of two layers of a thermoplastic material that enclose the swelling body and that are welded along their margins by welding seams.

7. Compress of one of the claims 2 - 5 and of claim 6 characterised in that it is rectangular and that the welding seams are under an angle between 2 and 45 degree in respect to the stitch course and wale directions.

8. Compress of one of the preceding claims characterised in that the cover consists of a non-soaking and/or hydrophibised material.

9. Compress of one of the preceding claims characterised in that it comprises a deodorant auxiliary substance.

10. Compress of one of the preceding claims characterised in that the selling body (1) is arranged between two laminating layers (3) for guaranteeing a uniform distribution of the superabsorbent.

11. Compress of one of the preceding claims characterised in that it comprises several chambers divided by separating layers (8).

12. Compress of claim 11 characterised in that the separating layers can be made at least partially permeable by mechanical, chemical and/or thermal treatment.

## Revendications

1. Compresse pour pansement humide pour le traitement de plaies en milieu humide comportant un corps pouvant s'imbiber (1) placé dans une enveloppe (2) au moins partiellement perméable à l'humidité, le corps (l) contenant un super-absorbant pour emmagasiner un liquide et le dégorger de façon continue, ce super-absorbant présentant une consistance gélatineuse lorsqu'il est gorgé de liquide, caractérisée en ce que l'enveloppe (2) consiste au moins partiellement en un voile textile extensible de façon non-élastique en direction longitudinale, diagonale et transversale, de manière à ce qu'à l'état humide la compresse soit molle et plastiquement déformable.

2. Compresse selon la revendication 1, caractérisée en ce que l'enveloppe (2) consiste au moins partiellement en un tricot ou tissu à mailles.

3. Compresse selon la revendication 2, caractérisée en ce que l'enveloppe consiste en un tricot ou tissu à mailles de pur polypropylène.

4. Compresse selon la revendication 3, caractérisée en ce que le fil dont est fait le tricot ou le tissu à mailles est un multifil texturé par soufflage d'air.

5. Compresse selon la revendication 4, caractérisée en ce que le fil a une finesse de 170 dtex avec 80 fibrilles, ou allant jusqu'à 85 dtex avec 40 fibrilles.

6. Compresse selon une des revendications précédentes, caractérisée en ce que l'enveloppe (2) consiste en deux couches d'un matériau thermoplastique entourant le corps pouvant s'imbiber et réunies par soudure le long de leurs bords.

7. Compresse selon une des revendications 2 à 5 et la revendication 6, caractérisée en ce qu'elle est rectangulaire et que les soudures forment un angle compris entre 2° et 45° avec les rangées de mailles ou de fils du tissu à mailles, respectivement du tricot.

8. Compresse selon une des revendications précédentes, caractérisée en ce que l'enveloppe consiste en un matériau non-absorbant et/ou hydrophobe.

9. Compresse selon une des revendications précédentes, caractérisée en ce qu'elle contient un agent auxiliaire désodorisant.

10. Compresse selon une des revendications précédentes, caractérisée en ce que le corps pouvant s'imbiber (1) est disposé entre deux couches laminées (3) pour assurer une répartition régulière du super-absorbant.

11. Compresse selon une des revendications précédentes, caractérisée en ce qu'elle comporte plusieurs compartiments séparés par des cloisons intermédiaires (8).

12. Compresse selon la revendication 11, caractérisée en ce que les cloisons (8) sont prévues pour être rendues au moins partiellement perméables par action mécanique, chimique et/ou thermique.
